# FASCICULE DE BREVET EUROPEEN

(11) **EP 0 673 639 B1**
(45) Date de publication et mention de la délivrance du brevet: **07.06.2000**
(21) Numéro de dépôt: 95400264.8
(22) Date de dépôt: 08.02.1995
(51) Int. Cl.: A61K 7/06

(54) **Compositions cosmétiques détergentes et utilisations**
Kosmetische Tensid-Präparate und deren Verwendung
Detergent cosmetic compositions and their use

(30) Priorité: 22.03.1994 FR 9403329
(43) Date de publication de la demande: 27.09.1995
(73) Titulaire: L'OREAL, 75008 Paris (FR)
(72) Inventeur: Beauquey, Bernard, F-92110 Clichy (FR); Decoster, Sandrine, F-93800 Epinay Sur Seine (FR)
(74) Mandataire: Andral, Christophe André Louis

(56) Documents cités:
- EP-A- 0 331 833
- DE-A- 3 206 448
- DATABASE WPI Week 9351, Derwent Publications Ltd., London, GB; AN 93410766 & JP-A-5 310 539 (KAO) 22 Novembre 1993

## Description

La présente invention concerne de nouvelles compositions cosmétiques destinées au nettoyage des cheveux, du cuir chevelu et/ou de la peau, à base de tensioactifs détergents, de silicone et d'argile , ainsi que leur utilisation dans cette application cosmétique.

Pour le nettoyage des cheveux et/ou de la peau, l'utilisation de compositions cosmétiques détergentes ( shampooings ou gels-douche par exemple) contenant à la fois des agents tensioactifs à pouvoir lavant et un ou plusieurs agents de conditionnement est courante.

En effet, pour améliorer les propriétés cosmétiques des compositions détergentes, et plus particulièrement de celles qui sont appelées à être appliquées sur des cheveux sensibilisés (i.e. des cheveux qui se trouvent abîmés ou fragilisés notamment sous l'action chimique des agents atmosphériques et/ou de traitements capillaires tels que permanentes, teintures ou décolorations), il est souvent nécessaire d'introduire dans ces dernières des agents cosmétiques complémentaires comme par exemple des silicones, qui apportent alors aux cheveux traités une facilité de démêlage et de coiffage, ainsi qu'une douceur et une brillance nettement accrues.

Compte tenu du caractère insoluble de ces silicones dans les milieux aqueux utilisés dans les shampooings, on cherche à maintenir les silicones sous forme dispersée. Cette forme dispersée permet à la silicone de se déposer sur les cheveux ou sur la peau et de ne pas être totalement éliminée lors du rinçage. Il importe toutefois que la mise en suspension ne perturbe pas les propriétés détergentes et moussantes de la composition cosmétique.

Il existe à ce jour peu de moyens pour maintenir efficacement en suspension les silicones insolubles, car il s'agit là d'un problème difficile à résoudre ; à cet effet, on a déjà proposé l'utilisation des dérivés d'esters ou d'éthers à longue chaîne (agents nacrants) ou des polysaccharides tels que la gomme de xanthane (gélifiants). Cependant, les agents nacrants présentent des problèmes de cristallisation qui entraînent une évolution (augmentation) de la viscosité des compositions au cours du temps ; les agents gélifiant présentent également des inconvénients, à savoir, d'une part que la mousse des compositions détergentes contenant de la gomme de xanthane (EP 326 272) se développe difficilement (mauvais démarrage de mousse) et que, d'autre part, les compositions n'ont pas une texture lisse et s'écoulent par paquets, ce qui est peu apprécié des utilisateurs.

J05-310539 décrit des compositions détergentes contenant (a) un agent tensioactif, (b)une huile de silicone, (c) de fines particules insolubles dans l'eau et (d) un polymère non ionique hydrosoluble choisi parmi les alcools polyvinyliques et les dérivés de cellulose.

La présente invention a pour but de proposer un nouveau moyen permettant de maintenir en suspension des silicones insolubles dans des shampooings.

Ainsi, à la suite d'importantes recherches menées sur la question, la demanderesse a maintenant découvert qu'en introduisant une argile dans les compositions cosmétiques détergentes à base de tensioactifs et de silicone, il est possible de maintenir en suspension lesdites silicones insolubles.

Les compositions selon l'invention sont stables : en particulier, il ne se produit aucun relargage de la silicone ou d'épaississement incontrôlé de la composition au cours du temps. En outre, les propriétés moussantes, telles que le démarrage de la mousse, ne sont pas diminuées. Les propriétés lavantes et les autres propriétés cosmétiques avantageuses (douceur, démêlage, coiffage) qui sont attachés à ces compositions sont par ailleurs conservées. Les compositions présentent enfin une texture non filante et fondante.

Toutes ces découvertes sont à la base de la présente invention.

Ainsi, selon la présente invention, il est maintenant proposé de nouvelles compositions cosmétiques détergentes, du type comprenant, dans un milieu aqueux cosmétiquement acceptable, au moins un agent tensioactif détergent et au moins une silicone insoluble, et qui sont caractérisées par le fait qu'elles comprennent en outre au moins une argile, lesdites compositions étant exemptes de gomme de xanthane et de polymères non ioniques hydrosolubles choisis parmi les alcools polyvinyliques et les dérivés de cellulose.

L'invention a également pour objet l'utilisation en cosmétique des compositions ci-dessus pour le nettoyage des cheveux, de la peau et/ou du cuir chevelu.

Elle a également pour objet l'utilisation d'une argile comme agent de mise en dispersion d'une silicone insoluble dans une composition cosmétique détergente contenant une telle silicone et au moins un agent tensioactif détergent.

D'autres caractéristiques, aspects et avantages de l'invention apparaîtront encore plus clairement à la lecture de la description qui va suivre, ainsi que des exemples concrets, mais nullement limitatifs, destinés à l'illustrer.

La nature des tensioactifs rentrant dans les compositions détergentes selon l'invention n'est pas critique. Les agents tensioactifs détergents utilisables selon l'invention peuvent être de type anionique, non ionique, amphotère, zwittérionique ou cationique. On utilise de préférence un ou plusieurs tensioactifs anioniques ou encore un mélange de tensioactifs anioniques et de tensioactifs amphotères, zwittérioniques ou non ioniques.

Ainsi, à titre d'exemple de tensioactifs anioniques utilisables, seuls ou mélanges, dans le cadre de la présente invention, on peut citer notamment les sels (en particulier sels alcalins, notamment de sodium, sels d'ammonium, sels d'amines, sels d'aminoalcools ou sels de magnésium) des composés suivants : les alkylsulfates, les alkyléthersulfates, les alkylamidoéthersulfates, les monoglycérides sulfates, les alkylglycérylsulfonates; les alkylsulfonates, les alkylphosphates, les alkylamidesulfonates, les alkylarylsulfonates, les α-oléfinesulfonates ; les alkylsulfosuccinates, les alkyléthersulfosuccinates, les alkylamidesulfosuccinates; les alkylsulfosuccinamates ; les alkylsulfoacétates les alkylétherphosphates; les acyliséthionates, les N-acylaminoacides tels que les N-acylsarcosinates, les N-acylglutamates et les N-acyltaurates,. Parmi les tensioactifs anioniques encore utilisables, on peut également citer les sels d'acides gras tels que les sels des acides undécylénique, oléique, ricinoléique, palmitique, stéarique, les acides d'huile de coprah ou d'huile de coprah hydrogénée les acylhydroxyacides tels que les acyl-lactylates. On peut également utiliser des tensioactifs faiblement anioniques, comme les acides alkyl-D-galactoside uroniques et leurs sels ainsi que les acides éthers carboxyliques polyoxyalkylénés, en particulier ceux comportant de 2 à 24 groupements oxyde d'éthylène, et leurs mélanges. (le radical alkyle ou acyle de tous ces différents composés comportant de préférence de 8 à 22 atomes de carbone).

A titre de tensioactifs non ioniques, on peut citer les alcools, les alphadiols, les alkylphénols ou les acides gras polyéthoxylés, polypropoxylés ou polyglycérolés, ayant une chaîne grasse comportant de 8 à 28 atomes de carbone, le nombre de groupements d'oxyde d'éthylène ou de propylène pouvant aller de 2 à 50 et celui de glycérol notamment de 2 à 30. On peut également citer les copolymères d'oxyde d'éthylène et de propylène, les condensats d'oxyde d'éthylène et de propylène sur des alcools gras; les amines ou les amides gras polyéthoxylés ayant de préférence de 2 à 30 moles d'oxyde d'éthylène, les amides gras polyglycérolés comportant en moyenne 1 à 5 groupements glycérol, les diglycolamides polyglycérolés, les esters d'acides gras du sorbitan éventuellement oxyéthylénés, les esters d'acides gras du sucrose, les esters d'acides gras polyoxyalkylénés, les alkylpolyglycosides éventuellement oxyalkylénés, les esters d'alkylglucosides, les dérivés de N-alkylglucamine et de N-acylméthylglucamines, les oxydes d'amine.

A titre de tensioactifs amphotères ou zwittérioniques, on peut citer les dérivés d'amines secondaires ou tertiaires aliphatiques, dans lesquels le radical aliphatique est une chaîne linéaire ou ramifiée comportant 8 à 22 atomes de carbone et contenant au moins un groupe anionique hydrosolubilisant (par exemple carboxylate, sulfonate, sulfate, phosphate ou phosphonate) ; on peut citer les alkylbétaines, les alkyldiméthylbétaines, les alkylsulfobétaines, les alkylamidoalkylbétaines, les alkylamidoalkylsulfobétaines, les dérivés d'imidazoline tels que les dérivés d'amphocarboxyglycinate ou d'amphocarboxypropionate.

Le ou les tensioactifs sont généralement présents dans les compositions conformes à l'invention dans des proportions comprises entre 5 à 50 % en poids, de préférence de 5 à 20 % en poids, par rapport au poids total de la composition.

Les silicones insolubles utilisables dans le cadre de la présente invention peuvent être choisis parmi toutes celles déjà connus en soi comme améliorant les propriétés cosmétiques des cheveux traités par des compositions détergentes, à savoir notamment celles et ceux décrits dans les demandes de brevets EP-A- 0 181773 et EP-A- 0 473508.

Il est bien entendu possible de mettre en oeuvre des mélanges de silicones.

Ainsi, selon la présente invention, il est possible d'utiliser toute silicone connue en soi, qu'il s'agisse d'une huile, d'une résine ou bien encore d'une gomme de silicone. Les silicones sont des polymères ou oligomères organosiliciés à structure linéaire ou cyclique, ramifiée ou réticulée, de poids moléculaire variable, obtenus par polymérisation et/ou polycondensation de silanes convenablement fonctionnalisés, et constitués pour l'essentiel par une répétition de motifs principaux dans lesquels les atomes de silicium sont reliés entre eux par des atomes d'oxygène (liaison siloxane), des radicaux hydrocarbonés éventuellement substitués étant directement liés par l'intermédiaire d'un atome de carbone sur lesdits atomes de silicium. Les radicaux hydrocarbonés les plus courants sont les radicaux alkyles et en particulier méthyle, les radicaux fluoroalkyles, les radicaux aryles et en particulier phényle, et les radicaux alcényles et en particulier vinyle; d'autres types de radicaux susceptibles d'être liés soit directement, soit par l'intermédiaire d'un radical hydrocarboné, à la chaîne siloxanique sont notamment l'hydrogène, les halogènes et en particulier le chlore, le brome ou le fluor, les thiols, les radicaux alcoxy, les radicaux polyoxyalkylènes (ou polyéthers) et en particulier polyoxyéthylène et/ou polyoxypropylène, les radicaux hydroxyles ou hydroxyalkyles, les groupements aminés substitués ou non, les groupements amides, les radicaux acyloxy ou acyloxyalkyles, les radicaux hydroxyalcylamino ou aminoalkyles, des groupements ammonium quaternaires, des groupements amphotères ou bétaïniques, des groupements anioniques tels que carboxylates, thioglycolates, sulfosuccinates, thiosulfates, phosphates et sulfates, cette liste n'étant bien entendu nullement limitative (silicones dites "organomodifiées"). D'une manière générale, les silicones utilisables dans le cadre de la présente invention sont celles qui sont notamment décrites dans "Encyclopedia of Chemical Technology, Kirk-Othmer, Third Edition, 1982, volume 20, pp. 922 et suivantes" et dans "Chemistry and Technology of Silicones, Walter NOLL, Academic Press Inc, San Diego California, 1968". Il est également possible d'utiliser des copolymères blocs linéaires comprenant dans leur chaîne principale des segment polysiloxaniques, comme par exemple des copolymères blocs polysiloxane-polyoxyalkylène ou bien encore polysiloxane-polyuréthane et/ou polyurée. Le poids moléculaire moyen des silicones utilisables selon l'invention peut varier entre 100 et plusieurs millions, de préférence entre 1000 et 1 000 000. Selon la présente invention, on peut bien entendu soit utiliser une seule et même silicone soit, mettre en oeuvre plusieurs silicones différentes.

A titre d'exemples de silicones utilisables dans les compositions détergentes selon l'invention, on peut notamment citer les polydialkylsiloxanes, les polyalkylarylsiloxanes, les polydiaryldialkylsiloxanes et d'une manière encore plus générale tous les organopolysiloxanes décrits dans la demande de brevet publiée sous le numéro WO 93/05762 et dont l'enseignement est, à cet égard, totalement inclus dans la présente demande à titre de référence.

Selon un mode de réalisation particulièrement préféré de la présente invention, les silicones utilisées sont choisies parmi les diorganopolysiloxanes (huiles, gommes ou résines), de préférence les polydialkylsiloxanes ou les polyalkylarylsiloxanes, et encore plus préférentiellement les polydiméthylsiloxanes éventuellement modifiés.

Les gommes de silicone sont particulièrement préférées et en particulier celles de polydialkylsiloxanes ou de polyalkylarylsiloxanes, éventuellement modifiés. Elles peuvent être utilisées seules ou en mélange dans un solvant choisi par exemple parmi les silicones volatiles, les huiles polydiméthylsiloxane ou polyphénylméthylsiloxane, les isoparaffines, le pentane, le dodécane ou leurs mélanges.

La ou les silicones sont présentes dans les compositions conformes à l'invention dans des proportions généralement comprises entre 0,01 à 10 % en poids, de préférence de 0,5 à 5 % en poids, par rapport au poids total de la composition.

Selon une caractéristique essentielle des compositions cosmétiques détergentes selon l'invention, celles-ci contiennent au moins une argile, naturelle ou synthétique, mais de préférence naturelle.

Les compositions détergentes conformes à l'invention peuvent bien entendu contenir une argile ou un mélange d'argiles.

Les argiles sont des produits déjà bien connus en soi qui sont décrits par exemple dans l'ouvrage "Minéralogie des argiles, S. Caillère, S. Hénin, M. Rautureau, 2ème édition 1982, Masson", dont l'enseignement est ici inclus à titre de référence.

A titre d'exemples de tels produits, on peut citer les argiles de la famille de la kaolinite telles que la kaolinite, la dickite, la nacrite, les argiles de la famille de l'halloysite, de la dombasite, de l'antigorite, de la berthiérine, de la pyrophyllite, des montmorillonites, de la beidellite, des vermiculites, du talc, de la stévensite, des hectorites, des saponites, des chlorites, de la sépiolite et leurs mélanges.

Les argiles peuvent également être modifiées chimiquement par divers composés tels que les acides acryliques, les polysaccharides (par exemple la carboxyméthylcellulose) ou les cations organiques.

De préférence, on retiendra les argiles qui sont cosmétiquement compatibles et acceptables avec les cheveux, la peau et/ou le cuir chevelu.

On utilise particulièrement les mélanges d'argiles de familles différentes.

Selon un mode particulièrement préféré de réalisation de la présente invention, l'argile mise en oeuvre est choisie parmi la kaolinite, les montmorillonites, les hectorites et leurs mélanges.

On utilise encore plus particulièrement les mélanges d'au moins une kaolinite et d'au moins une montmorillonite.

Lorsque l'on utilise un mélange de kaolinites et de montmorillonites, le rapport en poids kaolinites/montmorillonites peut varier de 0,75 à 10 et de préférence de 0,75 à 3.

Selon l'invention, la ou les argiles sont présentes dans les compositions généralement à raison de 0,5 à 15% en poids, et particulièrement de 2 à 10 % en poids, encore plus préférentiellement de 4 à 8% en poids, par rapport à l'ensemble de la composition.

Le véhicule, ou support, des compositions détergentes selon l'invention est de préférence de l'eau ou une solution hydroalcoolique par exemple d'un alcool inférieur tel que l'éthanol, l'isopropanol ou le butanol.

Les compositions détergentes selon l'invention présentent un pH final généralement compris entre 4 et 8. De préférence, ce pH est compris entre 5 et 7. L'ajustement du pH à la valeur désirée peut se faire classiquement par ajout, selon les cas, soit d'agents basifiants, soit d'agents acidifiants, usuels et connus comme cosmétiquement acceptables.

Les compositions détergentes selon l'invention peuvent bien entendu contenir en outre tous les adjuvants habituellement rencontrés dans le domaine des shampooings pour les cheveux et/ou pour le corps, comme par exemple des parfums, des agents conservateurs, des séquestrants, des agents acidifiants, des agents alcalinisants, des épaississants autres que l'argile, des adoucissants, des modificateurs de mousse, des colorants, des agents nacrants, des agents hydratants, des agents antipelliculaires ou anti-séborrhéiques, des vitamines, des filtres solaires, des polymères de préférence cationiques ou amphotères et autres.

Ces compositions peuvent se présenter sous la forme de liquides plus ou moins épaissis, de crèmes ou de gels et elles conviennent principalement au lavage des cheveux et/ou de la peau.

Des exemples concrets illustrant l'invention vont maintenant être donnés.

### EXEMPLE 1

On a préparé un shampooing conforme à l'invention qui présentait la composition suivante :
- Lauryl éther sulfate de sodium à 2,2 moles d'oxyde d'éthylène 9,8 g
- Cocoamidoéthyl (N-hydroxyéthyl, N-carboxyméthyl) glycinate de sodium, vendu sous le nom de MIRANOL C2M par RHONE-POULENC 3,8 g
- Polydiméthylsiloxane(PDMS)(Huile 47 V 500.000 RHONE POULENC) 1 g
- Kaolinite
   (KAOLIN SUPREME 1 de ETS ERNEST ORTMANS) 7g
- Conservateurs, colorants, parfum qs
- Eau qsp 100 g

Le pH de ce shampooing a été ajusté à pH 5,5 par ajout d'acide citrique.

Cette composition de shampooing est stable, les silicones sont parfaitement maintenues en suspension et la viscosité n'évolue pas au cours du temps. On applique cette composition sur des cheveux mouillés, on masse les cheveux, la mousse se développe immédiatement contrairement à une composition renfermant 1% de gomme de xanthane à la place de l'argile.

### EXEMPLE 2

On a préparé un shampooing conforme à l'invention qui présentait la composition suivante :
- Lauryl éther sulfate de sodium à 2,2 moles d'oxyde d'éthylène 9,8 g
- Cocoylbétaïne 2,24 g
- Mélange (13/87) d'une gomme PDMS et de cyclométhicone (Q2-1401 de DOW CORNING) 2,5 g
- Montmorillonite
   (GELWHITE HNF de ECC INTERNATIONAL) 4 g
- Copolymère acrylate d'alkyle (C₁₀/C₃₀) / acide acrylique réticulé (CARBOPOL 1382 de GOODRICH) 0,2 g
- Conservateurs, colorants, parfum qs
- Eau qsp 100 g

Le pH de ce shampooing a été ajusté à pH 5 par ajout d'acide citrique.

Cette composition de shampooing présente les mêmes propriétés que celle de l'exemple 1.

### EXEMPLE 3

On a préparé un shampooing conforme à l'invention qui présentait la composition suivante :
- Lauryl éther sulfate de sodium à 2,2 moles d'oxyde d'éthylène 9,8 g MA
- Cocoamidoéthyl (N-hydroxyéthyl, N-carboxyméthyl) glycinate de sodium, vendu sous le nom de MIRANOL C2M SF par RHONE-POULENC 3,2 g MA
- Mélange d'un polydiméthylsiloxane (PDMS) de PM 500 000 et d'un PDMS de viscosité 1000 Cst (33/67) vendu sous le nom de Silicone CF 1241 par la société GENERAL ELECTRIC 3 g
- Montmorillonite
   (GELWHITE HNF de ECC INTERNATIONAL 4 g
- Kaolinite
   (KAOLIN SUPREME 1 de ETS ERNEST ORTMANS) 5 g
- Conservateurs qsp
- Eau qsp 100 g

Le pH de ce shampooing a été ajusté à pH 5 par ajout d'acide citrique.

Cette composition de shampooing présente les mêmes propriétés que celle de l'exemple 1.

## Revendications

1. Compositions cosmétiques détergentes comprenant, dans un milieu aqueux cosmétiquement acceptable, au moins un agent tensioactif détergent et au moins une silicone insoluble, caractérisées par le fait qu'elles comprennent en outre au moins une argile, lesdites compositions étant exemptes de gomme de xanthane et de polymères non ioniques hydrosolubles choisis parmi les alcools polyvinyliques et les dérivés de cellulose.

2. Compositions selon la revendication 1, caractérisées par le fait que la teneur en argile est comprise entre 0,5 et 15% en poids par rapport au poids total de la composition.

3. Compositions selon la revendication 2, caractérisées par le fait que ladite teneur est comprise entre 2 et 10% en poids par rapport au poids total de la composition.

4. Compositions selon la revendication 3, caractérisées par le fait que ladite teneur en argile est comprise entre 4 et 8% en poids par rapport au poids total de la composition.

5. Compositions selon l'une quelconque des revendications précédentes, caractérisées par le fait que le ou les tensioactifs détergents sont présents à raison de 5 à 50% en poids, de préférence de 5 à 20 % en poids, par rapport au poids total de la composition.

6. Compositions selon l'une quelconque des revendications précédentes, caractérisées par le fait que la ou les silicones sont présentes à raison de 0,01 à 10 % en poids par rapport au poids total de la composition.

7. Compositions selon la revendication 6, caractérisées par le fait que la ou les silicones sont présentes à raison de 0,5 à 5 % en poids par rapport au poids total de la composition.

8. Compositions selon l'une quelconque des revendications précédentes, caractérisées par le fait qu'il s'agit de compositions aqueuses ou hydroalcooliques.

9. Compositions selon l'une quelconque des revendications précédentes, caractérisées par le fait qu'elles présentent un pH compris entre 4 et 8.

10. Compositions selon l'une quelconque des revendications précédentes, caractérisées par le fait que le ou les agents tensioactifs détergents sont de type anionique, non ionique, amphotère, zwittérionique ou cationique.

11. Compositions selon l'une quelconque des revendications précédentes, caractérisées par le fait que l'argile est choisie parmi les argiles de la famille de la kaolinite, de l'halloysite, de la dombasite, de l'antigorite, de la berthiérine, de la pyrophyllite, des montmorillonites, de la beidellite, des vermiculites, du talc, de la stévensite, des hectorites, des saponites, des chlorites, de la sépiolite et leurs mélanges.

12. Compositions selon la revendication 11, caractérisées par le fait que l'argile est choisie parmi la kaolinite, les montmorillonites, les hectorites et leurs mélanges.

13. Compositions selon l'une quelconque des revendications précédentes, caractérisées par le fait qu'elles comprennent un mélange d'argiles.

14. Compositions selon l'une quelconque des revendications précédentes, caractérisées par le fait qu'elles comprennent au moins une kaolinite et au moins une montmorillonite.

15. Compositions selon l'une quelconque des revendications précédentes, caractérisées par le fait que la silicone insoluble est choisie parmi les huiles de silicone, les gommes de silicone et les résines de silicone.

16. Compositions selon la revendication 15, caractérisées par le fait que la silicone insoluble est choisie parmi les polydialkylsiloxanes ou les polyalkylarylsiloxanes, éventuellement modifiés.

17. Compositions selon la revendication 15, caractérisées par le fait que la silicone insoluble est choisie parmi les gommes de polydialkylsiloxanes ou de polyalkylarylsiloxane, éventuellement modifiés.

18. Utilisation d'une argile comme agent de mise en dispersion d'une silicone insoluble dans une composition cosmétique détergente contenant une silicone et au moins un agent tensioactif détergent.

19. Utilisation d'une composition telle que définie à l'une quelconque des revendications 1 à 17 pour le nettoyage des cheveux, du cuir chevelu et/ou de la peau.

## Patentansprüche

1. Reinigende kosmetische Zusammensetzungen, die in einem kosmetisch akzeptablen Medium mindestens einen reinigenden grenzflächenaktiven Stoff und mindestens ein unlösliches Silicon enthalten,
dadurch gekennzeichnet, daß
sie ferner mindestens einen Ton enthalten, wobei die Zusammensetzungen kein Xanthangummi und keine nichtionischen wasserlöslichen Polymere, die unter den Polyvinylalkoholen und Cellulosederivaten ausgewählt sind, enthalten.

2. Zusammensetzungen nach Anspruch 1, dadurch gekennzeichnet, daß der Tongehalt im Bereich von 0,5 bis 15 Gew.-%, bezogen auf das Gesamtgewicht der Zusammensetzung, liegt.

3. Zusammensetzungen nach Anspruch 2, dadurch gekennzeichnet, daß der Gehalt im Bereich von 2 bis 10 Gew.-%, bezogen auf das Gesamtgewicht der Zusammensetzung, liegt.

4. Zusammensetzung nach Anspruch 3, dadurch gekennzeichnet, daß der Tongehalt im Bereich von 4 bis 8 Gew.-%, bezogen auf das Gesamtgewicht der Zusammensetzung, liegt.

5. Zusammensetzungen nach einem der vorhergehenden Ansprüche, dadurch gekennzeichnet, daß der (die) grenzflächenaktive(n) Stoff(e) in einem Mengenanteil im Bereich von 5 bis 50 Gew.-% und vorzugsweise 5 bis 20 Gew.-%, bezogen auf das Gesamtgewicht der Zusammensetzung, vorliegen.

6. Zusammensetzungen nach einem der vorhergehenden Ansprüche, dadurch gekennzeichnet, daß das Silicon oder die Silicone in einem Mengenanteil im Bereich von 0,01 bis 10 Gew.-%, bezogen auf das Gesamtgewicht der Zusammensetzung, vorliegen.

7. Zusammensetzungen nach Anspruch 6, dadurch gekennzeichnet, daß das Silicon oder die Silicone in einem Mengenanteil von 0,5 bis 5 Gew.-%, bezogen auf das Gesamtgewicht der Zusammensetzung, vorliegen.

8. Zusammensetzungen nach einem der vorhergehenden Ansprüche, dadurch gekennzeichnet, daß es sich um wäßrige oder wäßrig-alkoholische Zusammensetzungen handelt.

9. Zusammensetzungen nach einem der vorhergehenden Ansprüche, dadurch gekennzeichnet, daß sie einen pH-Wert im Bereich von 4 bis 8 aufweisen.

10. Zusammensetzungen nach einem der vorhergehenden Ansprüche, dadurch gekennzeichnet, daß der (die) reinigende(n) grenzflächenaktive(n) Stoff(e) vom anionischen, nichtionischen, amphoteren, zwitterionischen oder kationischen Typ sind.

11. Zusammensetzungen nach einem der vorhergehenden Ansprüche, dadurch gekennzeichnet, daß der Ton unter den Tonen der folgenden Gruppen ausgewählt ist: Kaolinit, Halloysit, Donbassit, Antigorit, Berthierin, Pyrophyllit, Montmorilloniten, Beidellit, Vermiculiten, Talk, Stevensit, Hectoriten, Saponiten, Chloriten, Sepiolit und deren Gemischen.

12. Zusammensetzungen nach Anspruch 11, dadurch gekennzeichnet, daß der Ton unter Kaolinit, Montmorilloniten, Hectoriten und deren Gemischen ausgewählt ist.

13. Zusammensetzungen nach einem der vorhergehenden Ansprüche, dadurch gekennzeichnet, daß sie ein Gemisch von Tonen enthalten.

14. Zusammensetzungen nach einem der vorhergehenden Ansprüche, dadurch gekennzeichnet, daß sie mindestens einen Kaolinit und mindestens einen Montmorillonit enthalten.

15. Zusammensetzungen nach einem der vorhergehenden Ansprüche, dadurch gekennzeichnet, daß das unlösliche Silicon unter den Siliconölen, Silicongummis und Siliconharzen ausgewählt ist.

16. Zusammensetzungen nach Anspruch 15, dadurch gekennzeichnet, daß das unlösliche Silicon unter den Polydialkylsiloxanen oder Polyalkylarylsiloxanen, die gegebenenfalls modifiziert sind, ausgewählt ist.

17. Zusammensetzungen nach Anspruch 15, dadurch gekennzeichnet, daß das unlösliche Silicon unter den Polydialkylsiloxangummis oder Polyalkylarylsiloxangummis, die gegebenenfalls modifiziert sind, ausgewählt ist.

18. Verwendung eines Tons als Mittel zum Dispergieren für ein Silicon, das in einer reinigenden kosmetischen Zusammensetzung, die ein Silicon und mindestens einen reinigenden grenzflächenaktiven Stoff enthält, unlöslich ist.

19. Verwendung einer Zusammensetzung nach einem der Ansprüche 1 bis 17 zur Reinigung der Haare, der Kopfhaut und/oder der Haut.

## Claims

1. Detergent cosmetic compositions comprising, in a cosmetically acceptable aqueous medium, at least one detergent surfactant and at least one insoluble silicone, characterized in that they also comprise at least one clay, the said compositions being free of xanthan gum and of water-soluble nonionic polymers chosen from polyvinyl alcohols and cellulose derivatives.

2. Compositions according to Claim 1, characterized in that the clay content is between 0.5 and 15% by weight relative to the total weight of the composition.

3. Compositions according to Claim 2, characterized in that the said content is between 2 and 10% by weight relative to the total weight of the composition.

4. Compositions according to Claim 3, characterized in that the said clay content is between 4 and 8% by weight relative to the total weight of the composition.

5. Compositions according to any one of the preceding claims, characterized in that the detergent surfactant(s) is(are) present in a proportion of from 5 to 50% by weight, preferably from 5 to 20% by weight, relative to the total weight of the composition.

6. Compositions according to any one of the preceding claims, characterized in that the silicone(s) is(are) present in a proportion of from 0.01 to 10% by weight relative to the total weight of the composition.

7. Compositions according to Claim 6, characterized in that the silicone(s) is(are) present in a proportion of from 0.5 to 5% by weight relative to the total weight of the composition.

8. Compositions according to any one of the preceding claims, characterized in that they are aqueous or aqueous-alcoholic compositions.

9. Compositions according to any one of the preceding claims, characterized in that they have a pH of between 4 and 8.

10. Compositions according to any one of the preceding claims, characterized in that the detergent surfactant(s) is(are) of anionic, nonionic, amphoteric, zwitterionic or cationic type.

11. Compositions according to any one of the preceding claims, characterized in that the clay is chosen from clays of the kaolinite, halloysite, dombasite, antigorite, berthierine, pyrophyllite, montmorillonite, beidellite, vermiculite, talc, stevensite, hectorite, saponite, chlorite or sepiolite family, and mixtures thereof.

12. Compositions according to Claim 11, characterized in that the clay is chosen from kaolinite, montmorillonites, hectorites and mixtures thereof.

13. Compositions according to any one of the preceding claims, characterized in that they comprise a mixture of clays.

14. Compositions according to any one of the preceding claims, characterized in that they comprise at least one kaolinite and at least one montmorillonite.

15. Compositions according to any one of the preceding claims, characterized in that the insoluble silicone is chosen from silicone oils, silicone gums and silicone resins.

16. Compositions according to Claim 15, characterized in that the insoluble silicone is chosen from polydialkylsiloxanes or polyalkylarylsiloxanes, which are optionally modified.

17. Compositions according to Claim 15, characterized in that the insoluble silicone is chosen from polydialkylsiloxane or polyalkylarylsiloxane gums, which are optionally modified.

18. Use of a clay as an agent for dispersing an insoluble silicone in a detergent cosmetic composition containing a silicone and at least one detergent surfactant.

19. Use of a composition as defined in any one of Claims 1 to 17, for cleaning the hair, the scalp and/or the skin.
